# EUROPEAN PATENT SPECIFICATION

(11) **EP 4 387 486 B1**
(45) Date of publication and mention of the grant of the patent: **19.11.2025**
(21) Application number: 22751765.3
(22) Date of filing: 09.08.2022
(51) Int. Cl.: A45D 26/00, A45D 34/04, A61M 35/00, A61M 11/00

(54) **A SKIN TREATMENT DEVICE**
HAUTBEHANDLUNGSVORRICHTUNG
DISPOSITIF DE TRAITEMENT DE LA PEAU

(30) Priority: 18.08.2021 EP 21192008
(43) Date of publication of application: 26.06.2024
(73) Proprietor: Koninklijke Philips N.V., 5656 AG Eindhoven (NL)
(72) Inventor: KOENEN, Maurits, 5656AG Eindhoven (NL)
(74) Representative: Philips Intellectual Property & Standards
(86) International application number: PCT/EP2022/072297
(87) International publication number: WO 2023/020882

(56) References cited:
- EP-A1- 2 103 232
- WO-A1-2016/196915
- WO-A1-2017/121623
- US-A- 5 121 541

## Description

### TECHNICAL FIELD

The present disclosure generally relates to a device for performing a treatment operation on skin of a subject, and in particular to the skin treatment operation in which the operation of the skin treatment device is controlled based on operating parameters.

### BACKGROUND

For aesthetic or personal reasons, many people find it desirable to treat their skin by removing unwanted hair from various areas of the human body. Various techniques for removal of unwanted hair includes shaving, electrolysis, plucking, laser therapies and injection of therapeutic antiandrogens. Usually, unwanted hair is removed by plucking and various devices are adapted for such hair removing technique. However, such devices pose a fundamental drawback in that the plucking of the hair is painful. This limits widespread use of such devices, particularly for sensitive skin surfaces.

Considering the above, hair removal devices equipped with cooling function have evolved. Such hair removal devices are structed to cool the skin prior to removal of hair. However, such devices tend to excessively cool the skin, which dampens the skin and, thus results inefficient hair removal, which is undesired. Moreover, in typical skin treatment devices with integrated cooling mechanics, the cooling action can either be induced continuously or by initiation of the user. These options are relatively less efficient and/or effective. For example, a user/subject may be less sensitive to pain caused by mechanical epilation. In this case, a continuous or increased cooling of skin leads to an inefficient use of battery power. On the other hand, allowing the user/subject to manually control the function may lead to cases where cooling is not initiated when needed, resulting in reduced pain reduction.

Further, currently available hair removal devices require user to position the device over an area of skin, move the device to another area of skin, until the user or subject considers the portion of the skin has been adequately treated, with all components (such as cooling delivery components) of the hair removal device, being fully operational throughout the hair removal operation. The user/subject does not always operate the device with the correct speed. When used faster than the desired speed, the treatment can be incomplete. When used slower (e.g. repeatedly over the same area), the skin can overheat and cause irritation. As a result of this, users of the hair removal devices face difficulties in efficiently and effectively using such devices over large areas of the skin. These difficulties arise from a combination of relatively large area of skin to be treated, lack of information about correct usage of the hair removal device, lack of information on parameters of the skin subjected to treatment and recommended treatment time for specific location of the body. These user-dependent issues result in high variability in compliance, treatment time and ultimately treatment efficacy.

Present disclosure is directed to overcome one or more limitations stated above or any other limitations associated with the devices known in art.

WO 2016/196915 A1 discloses a device for delivering fluid to a surface containing cells. In one embodiment, a fluid delivery device is provided that includes a housing, a fluid vaporizer disposed within the housing and configured to receive fluid and to produce an aerosol mist of liquid particles from the fluid, a trigger which triggers the fluid vaporizer and a pump configured to accelerate the aerosol mist produced by the fluid vaporizer.

EP 2 103 232 A1 discloses an epilator including a vaporizer unit 10 which vaporizes an application material 50. It may further include a cooling unit 13 or heating unit 12 which cools or warms the application material, to intensity its effect on skin.

### SUMMARY OF THE DISCLOSURE

It is an objective to provide a skin treatment device for performing treatment operation on skin by (automatically) controlling the cooling effect to the treated skin and/or controlling operational speed of a skin treatment tool, based on detected operating parameters, to enable the user to complete the treatment operation in an efficient and effective manner. It would be desirable to enable the skin treatment device to control the cooling effect and/or operational speed of the skin treatment tool based on operating parameters such as skin parameters (as non-limiting examples, temperature and humidity of the skin, and skin tone) and parameters of the skin treatment device (e.g., temperature of the skin treatment tool), for efficient operation of the skin treatment device. An effective way of cooling the skin is through aerosol generation. Although other methods such as Peltier cooling exist in the context of skin treatment devices, these are more expensive.

To better address one or more of these concerns, in a first aspect of the disclosure there is provided a skin treatment device. The skin treatment device includes a head, an aerosol generating device and an operating parameter sensor. The aerosol generating device is arranged to generate and channelize an aerosol through a flow channel of the skin treatment device out of the skin treatment device and onto skin subjected to treatment to provide a cooling effect to the skin, based on an operating parameter measured by the operating parameter sensor. The aerosol generating device is disposed on the head and parallel to a longitudinal axis of the skin treatment device.

In an embodiment, the skin treatment device further includes a flow stream generating element, configured to generate a flow to further channelize the aerosol through the flow channel.

In an embodiment, the flow stream generating element is at least one of a blower and a suction fan.

In an embodiment, the flow channel is defined by spacings in a skin treatment tool of the skin treatment device.

In an embodiment, the operating parameter sensor is an optical sensor.

In an embodiment, the operating parameter sensor is configured to measure at least one of a skin parameter and a skin treatment device parameter.

In an embodiment, the skin parameter is at least one of temperature of skin, humidity of skin, and skin tone.

In an embodiment, the skin treatment device parameter is temperature, mechanical force and/or displacement of a skin treatment tool.

In an embodiment, the aerosol generating device is a piezo-electric device.

In an embodiment, the skin treatment device further includes a plurality of operating parameter sensors. Each of the plurality of operating parameter sensors are disposed on opposite sides relative to a longitudinal axis of a skin treatment tool of the skin treatment device.

In an embodiment, the skin treatment device includes a control unit communicatively coupled to the operating parameter sensor and the aerosol generating device. The control unit is configured to selectively control operation of the aerosol generating device and/or a skin treatment tool of the skin treatment device based on the operating parameter measured by the operating parameter sensor.

In an embodiment, the control unit is further communicatively coupled to a flow stream generating element and configured to actuate the flow stream generating element independent of the aerosol generating device based on humidity of the skin measured by the operating parameter sensor.

In an embodiment, the skin treatment device includes an indication unit communicatively coupled to a control unit, wherein the control unit is configured to generate an alert signal through the indication unit based on signals from the operating parameter sensor, and/or wherein the alert signal is at least one of an audio signal, a visual signal, an audio-visual signal, and a haptic signal.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF ACCOMPANYING DRAWINGS

The invention is as set forth in the appended claims, while encompassing various modifications routine to the skilled person and claim equivalents. The disclosure itself, as well as a mode of use, further objectives, and advantages thereof, will best be understood by reference to the following detailed description of the embodiments when read in conjunction with the accompanying drawings. One or more embodiments are now described, by way of example only, with reference to the accompanying exemplary drawings wherein like reference numerals represent like elements and in which:
Fig. 1 illustrates a perspective view of a skin treatment device, in accordance with an embodiment of the present disclosure;
Fig. 2 illustrates an exploded view of the skin treatment device of Fig 1.
Fig. 3a illustrates a perspective view of a portion of the skin treatment device, in accordance with an embodiment of the present disclosure;
Fig. 3b illustrates a side view of a portion of the skin treatment device, in accordance with an embodiment of the present disclosure;
Fig. 4 is a block diagram illustrating components of the skin treatment device, in accordance with an embodiment of the present disclosure; and
Fig. 5 is a block diagram of a control unit controlling operations of the skin treatment device, in accordance with an embodiment of the present disclosure.

The figures depict embodiments of the disclosure for purposes of illustration only. One skilled in the art will readily recognize from the following description that alternative embodiments of the structures and methods illustrated herein may be employed without departing from the principles of the disclosure described herein.

### DETAILED DESCRIPTION

While embodiments in the disclosure are subject to various modifications and alternative forms, specific embodiments thereof have been shown by way of example in the figures and will be described below. It should be understood, however, that it is not intended to limit the disclosure to the particular forms disclosed, but on the contrary, the disclosure is to cover all modifications, equivalents, and alternatives falling within the scope of the disclosure as described in the appended claims.

It is to be noted that a person skilled in the art would be motivated from the present disclosure and modify various features of a skin treatment device. Therefore, such modifications are part of the disclosure. Accordingly, the drawings show only those specific details that are pertinent to understand the embodiments of the present disclosure, so as not to obscure the disclosure with details that will be readily apparent to those of ordinary skilled in the art having benefit of the description herein.

The terms "comprises", "comprising", or any other variations thereof used in the disclosure, are intended to cover a non-exclusive inclusions, such that an assembly or unit that comprises a list of components does not include only those components but may include other components not expressly listed or inherent to suchdevice. In other words, one or more elements in an device proceeded by "comprises... a" does not, without more constraints, preclude the existence of other elements or additional elements in the system or device.

In the following detailed description, embodiments of the disclosure are explained with reference to accompanying figures that form a part hereof, and which are shown by way of illustration and specific embodiments in which the disclosure may be practiced. These embodiments are described in sufficient detail to enable those skilled in the art to practice the disclosure, and it is to be understood that other embodiments may be utilized and that changes may be made without departing from the scope of the present disclosure as described in the appended claims. The following description is, therefore, not to be taken in a limiting sense.

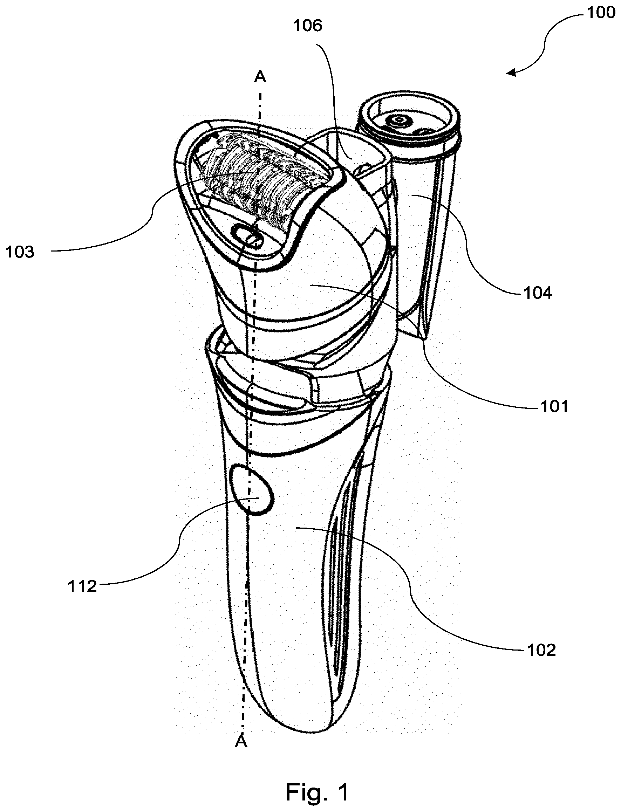

Fig. 1 illustrates a perspective view of a skin treatment device (100), in accordance with some embodiments of the present disclosure. The skin treatment device (100) may be a handheld device, which may be adapted for performing a treatment operation on a skin of a subject, by providing selective cooling effect to the treated skin. The term "treated skin" refers a portion of the skin subjected to treatment operation by the skin treatment device (100). In an embodiment, the treatment operation may be hair removal, hair reduction and the like. As described herein, the skin treatment device (100) is operated or used by a 'user', and the treatment operation is performed on a 'subject'. In some cases, the user and the subject is the same person, i.e., the skin treatment device (100) is held in a hand and used by the user on themselves (e.g., for treating the skin on their leg or hand and the like). In other cases, the user and the subject may be different, e.g., the skin treatment device (100) may be held in a hand and used by a user on someone else.

The skin treatment device (100) includes a head (101) and a handle (102), which may be removably connected to each other. In an embodiment, the head (101) and handle (102) may be removably connected to each other by a connection means including but not limiting a threaded connection, a snap fit connection and the like. The handle (102) may be configured to accommodate various components including a power source such as a battery, a driving unit [not shown in Figs] such as but not limiting to an electric motor, driving circuitry, a user control (112) and the like. Further, the skin treatment device (100) may include a skin treatment tool (103) which may be coupled to the driving unit. The skin treatment tool (103) may be rotateably disposed in or on the head (101), such that the skin treatment tool (103) may be placed adjacent to or on (i.e., in contact with) the skin of the subject. In an embodiment, the skin treatment tool (103) may include a plurality of rotating elements, which may contact the skin to perform treatment operation, when the skin treatment device (100) is traversed on the skin to be treated. The rotating elements act as tweezers pinching and extracting hairs by their rotating motion. The plurality of rotating elements may be made of suitable material such as but not limiting to polymer and metal.

As seen in Fig. 1, the skin treatment device (100) includes an aerosol generating device (104), which is disposed on the head (101) portion and may be electrically coupled to the power source of the skin treatment device (100). The aerosol generating device (104) may be configured to generate aerosol or mist from skin treatment fluids such as but not limiting to water. The aerosol generating device (104) may include an inlet and an outlet, defined at suitable positions, e.g., substantially opposite sides, of the aerosol generating device (104). In an illustrated embodiment, the aerosol generating device (104) is disposed on the head (101), parallel to a longitudinal axis (A-A) of the skin treatment device (100). In an embodiment, the skin treatment device (100) may include a reservoir [not shown in Figs] which may be positioned adjacent and fluidly coupled to the inlet of the aerosol generating device (104). In some embodiments, the aerosol generating device (104) may include the reservoir i.e., the reservoir may be an integral part of the aerosol generating device (104). The reservoir may store the fluid and supply fluid into the aerosol generating device (104). The fluid may enter the aerosol generating device (104) through the inlet and aerosol may be generated. The generated aerosol may exit through the outlet of the aerosol generating device (104). Thereafter, the generated aerosol is channelized out of the skin treatment device (100) and onto the treated skin, to provide cooling effect to the skin subjected to treatment.

The skin treatment device (100) may further include a flow channel (106), which may be coupled to the head (101). In some embodiments, the flow channel (106) may be sandwiched between the head (101) and the aerosol generating device (104) [as shown in Fig. 1] such that, the outlet of the aerosol generating device (104) is in fluid communication with the flow channel (106). In an embodiment, the flow channel (106) may be passage which may be integrally defined in the head (101) or may be externally coupled to the head (101). In an embodiment, the passage [thus, the flow channel (106)] may be a hollow structure having a substantially rectangular profile. However, the same cannot be construed as a limitation, since the hollow structure may have any other desired geometrical profiles such as but not limiting to circular, elliptical, square and any other polygonal shapes. In some embodiments, the flow channel (106) may include a tapered configuration, to facilitate effective channelizing of the aerosol onto the skin. In an embodiment, the flow channel (106) is a dedicated flow channel and includes a further outlet arranged to expel the aerosol out of the skin treatment device (100) onto the skin. In another embodiment, the flow channel may simply function to allow the flow to be channelized from the aerosol generating device (104) to the head (101) of the skin treatment device (100), for expulsion of the aerosol through another suitable outlet.

In an embodiment, the flow channel (106) may be defined by spacings in the skin treatment tool (103). That is, the flow channel (106) may be defined by spacings between the rotating elements of the skin treatment tool (103). The spacings may allow flow of the aerosol out of the skin treatment device (100) [thus, the head (101) of the skin treatment device (100)] and onto the skin, hence also serving as said further outlet. In this embodiment, the outlet of the aerosol generating device (104) may be positioned in fluid communication with the head (101). The aerosol exiting through the outlet of the aerosol generating device (104) may enter into the head (101), which may be then channelized through the spacings defined between the rotating elements of the skin treatment tool (103), onto the skin.

In an embodiment, the aerosol generating device (104) may be but not limiting to a piezo-electric device. The piezo-electric device may generate ultra-fine and high-volume mist or vapour particles from the fluid entering from the reservoir. The ultra fine mist or vapour generated by the piezo-electric device may be easily channelized out of the skin treatment device (100), thus facilitate effective cooling to the treated skin. The ultra-fine particle size of aerosols generated by the piezo-electric device further helps cool the skin without dampening it.

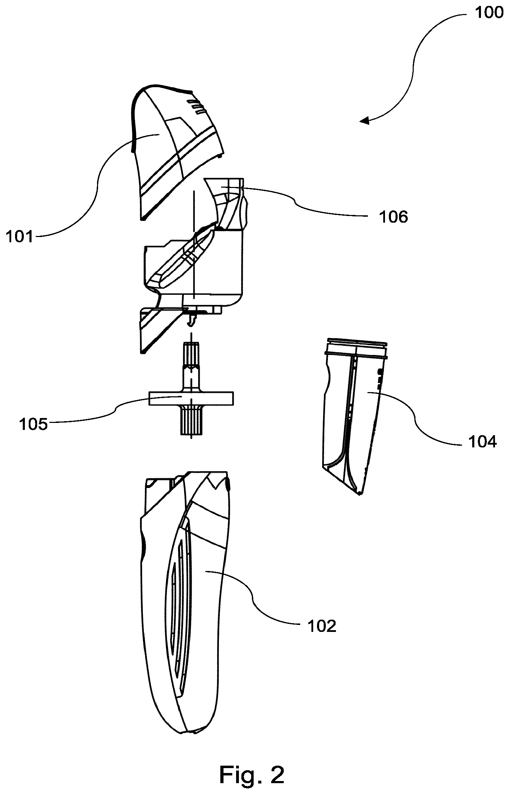

Referring to Fig. 2, the skin treatment device (100) may include a flow stream generating element (105) disposed within or adjacent to the head (101) and coupled to the driving unit. In an embodiment, the flow stream generating element (105) may be coupled to same driving unit to which the skin treatment tool (103) is coupled or may be coupled to a separate driving unit. The flow stream generating element (105) is configured to generate a fluid flow, in particular air flow to further channelize the generated aerosol through the flow channel (106) and onto the treated skin. That is, the flow stream generating element (105) may generate a stream of fluid to force the generated aerosol out of the skin treatment device (100) through the flow channel (106). The generated stream can be expelled out of the skin treatment device (100) either via the spacings of the rotating elements (103) or the dedicated flow channel (106) via the further outlet discussed above. In an embodiment, the flow stream generating element (105) may be at least one of a blower and a suction fan (not shown). The blower may be disposed at a lower position than the outlet of the aerosol generating device (104), such that the aerosol exiting through the outlet is forced or pushed through the flow channel (106), out of the skin treatment device (100) and onto the skin. The suction fan may be disposed at a higher position than the outlet of the aerosol generating device (104), such that the aerosol exiting through the outlet of the aerosol generating device (104) may be sucked through the flow channel (106), due to suction force or negative pressure generated by the suction fan.

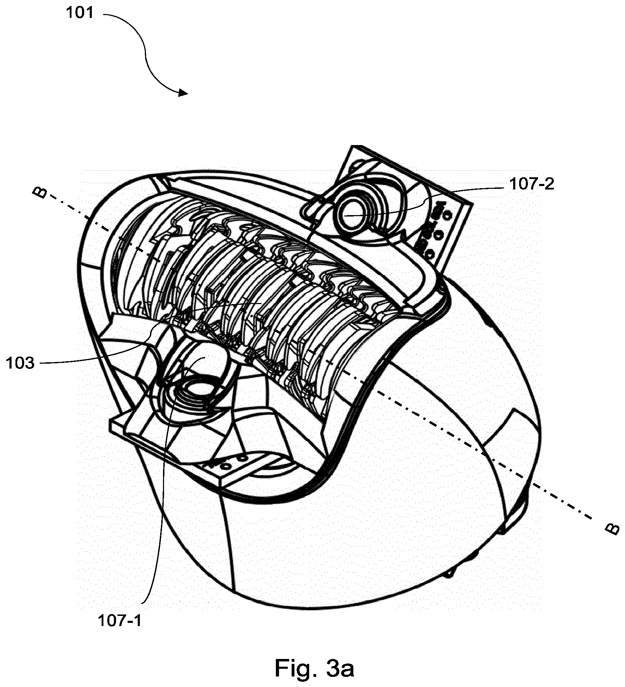

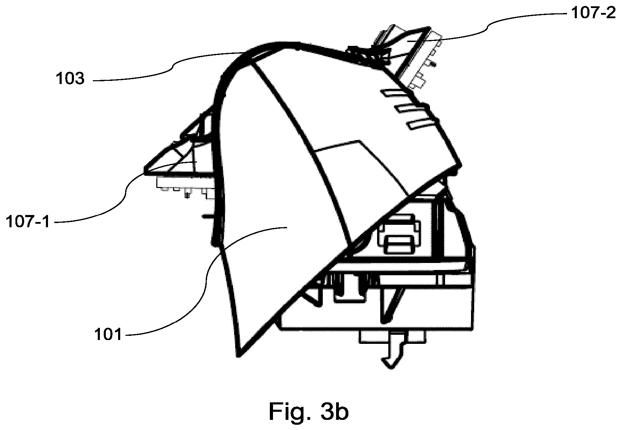

Turning now to Fig. 3, the skin treatment device (100) may include an operating parameter sensor (107), which may be disposed in or on the head (101). Although Fig. 3 shows two operating parameter sensors (107), it is possible to carry out the invention with a single sensor. The operating parameter sensor (107) may be configured to measure at least one of skin parameter and a skin treatment device parameter. In an embodiment, the skin parameter is at least one of temperature of skin (particularly the surface temperature of the skin), humidity of skin (particularly surface humidity of the skin) and skin tone/pigmentation. Further, the skin treatment device parameter is at least temperature of the skin treatment tool (103), mechanical force and/or displacement of the skin treatment tool (103). Fig. 3 shall now be explained with reference to the embodiment relating to temperature sensing.

In an embodiment the operating parameter sensor (107) may include one or more temperature sensors for measuring temperature of the skin and/or temperature of the skin treatment tool (103), before and after treatment of the skin. In an illustrated embodiment, as seen in Figs. 3a and 3b, a plurality of temperature measuring sensors such as but not limiting to non-contact temperature sensors (i.e., a sensor that does not require contact with the skin to measure the temperature of the skin), such as but not limiting to infrared (IR) thermal sensor, may be positioned adjacent to the skin treatment tool (103) and at opposite sides relative to a longitudinal axis (B-B) of the skin treatment tool (103) to obtain temperature measurements before and after skin treatment. The plurality of operating parameter sensors (thus, the IR sensors) may extend in a direction away from the head (101) and may be configured to measure temperature of a portion of skin and/or the skin treatment tool (103), before and after treatment of the skin. In Figs. 3a and 3b, sensor 107-1 measures the temperature of the skin at a location before it is treated. Sensor 107-2 measures the temperature at the same location after it is treated. Alternatively, each of the plurality of temperature sensors may be a contact temperature sensor (i.e., a sensor that requires contact with the skin to measure the temperature of the skin), such as a thermocouple, a thermistor, or a resistance temperature detector (RTD).

In other embodiments, the skin parameter may be an optical property of the skin, for example an optical property of the skin that changes in response to incident energy pulses. The optical property could be, e.g., scattering, reflectance, etc., and each operating parameter sensor (107) may be an optical sensor, which may be positioned on or in the head (101). The optical sensor may be configured to measure light reflected or scattered from the skin. In some embodiments, the optical sensors may be disposed on or in the head (101) along with an optical guide such as a mirror or a prism. The optical sensors may emit light into or onto the skin, with the optical guide, guiding the light emitted onto the skin. Further, the optical sensor measures the reflection or scattering of that light. Those skilled in the art will be aware of various techniques that can be used to measure the optical properties of the skin, including, for example, hyperspectral imaging, polarisation imaging and speckle imaging.

In other embodiments, the skin parameter may be an acoustic property of the skin, for example an acoustic property of the skin that changes in response to incident energy pulses. The acoustic property may be, e.g., acoustic impedance, speed of sound, etc., and the operating parameter sensor (107) may be an acoustic sensor, for example an ultrasound sensor. In some embodiments, the operating parameter sensor (107) may include a sound source for emitting sound into the skin, with the acoustic sensors measuring the sound to determine the acoustic skin property.

In other embodiments, the skin parameter may be an electrical property of the skin, for example an electrical property of the skin that changes in response to incident energy pulses. The electrical property could be, e.g., radio frequency (RF) impedance, capacitance, etc., and each operating parameter sensor (107) may be an RF sensor, an impedance sensor, or a capacitance sensor.

In another embodiment, the operating parameter sensor (107) includes one or more humidity sensors, which may be disposed in or on the head (101). The one or more humidity sensors may be configured to measure humidity of the skin, in particular surface humidity of the skin, as said skin parameter. As an example, the humidity sensors may be but not limiting to capacitive humidity sensor, resistive humidity sensor and thermal conductivity humidity sensor.

In other embodiments, the skin treatment device parameter may relate to force or tension exerted by the head (101) on the skin, or the distance moved by the head (101) and correspondingly time taken to complete the treatment action.

In an embodiment, the skin treatment device (100) may further include additional components to those illustrated in Figs. 1 to 3. The skin treatment device (100) may include a power source component (wire and plug) for enabling the skin treatment device (100) to be connected to mains power supply. Further, the skin treatment device (100) may include an indication unit [not shown in Figs], which is communicatively coupled to the control unit (108). An alert signal may be generated by the control unit (108) through the indication unit based on signals from the operating parameter sensor (107). The alert signal may be at least one of a visual indication (e.g. a light using a light source or visual signal on a display screen located either on head (101) or handle (102) of the skin treatment device (100) and visible to the user during use), an audio signal (e.g. a sound, such as a beep, using a sound source, e.g. a loudspeaker), an audio-video signal and a haptic signal (e.g. a vibration generated using a vibrating element located inside the body).

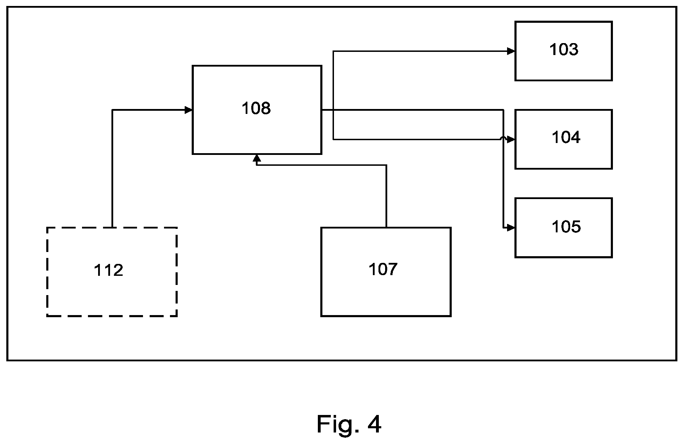

Turning now to Fig. 4, the skin treatment device (100) may include a control unit (108), which may be communicatively coupled to each of the operating parameter sensor (107), the aerosol generating device (104), the flow stream generating element (105) and the skin treatment tool (103). The control unit (108) may be configured to control operation of the skin treatment device (100). In an embodiment, controlling operation of the skin treatment device (100) may include controlling operation of the aerosol generating device (104) and the flow stream generating element (105), and the skin treatment tool (103) based on operating parameters (i.e., the skin parameters and the skin treatment tool (103) parameters), measured by the operating parameter sensor (107).

In an embodiment, "controlling operation" may correspond to adjusting operational capacity of at least one of the aerosol generating device (104), the flow stream generating element (105) and the skin treatment tool (103), depending on the determined operating parameters.

For example, the control unit (108) or the corresponding method obtains a difference of the sensor output signals of sensors 107-1 and 107-2 to calculate an increase in a skin parameter/skin treatment device parameter due to the treatment. Based on comparing the increase to a predetermined value, the control unit (108) may transmit a trigger signal to control the action of at least one of the aerosol generating device (104), the flow stream generating element (105) and the skin treatment tool (103). In case of implementing a single operational parameter sensor, an operational parameter measurement taken after treatment can be compared to a stored pre-treatment reference value.

In an embodiment, the control unit (108) or the corresponding method controls the action of the aerosol generating device (104) by transmitting a trigger signal to activate it, with or without activating the flow stream generating element (105). In another embodiment, the control unit (108) controls the action of the flow stream generating element (105) by transmitting a trigger signal to activate it, with or without activating the aerosol generating device (104). For example, for smaller difference above threshold, it suffices to induce the dry cooling action facilitated by the flow stream generating element (105). An activation of the flow stream generating element (105) independent of the aerosol generating device (104) further helps conserve battery power.

Correspondingly, if the increase is determined to be less than the predetermined threshold, the control unit (108) controls the action of the aerosol generating device (104) by transmitting a trigger signal to stop or reduce the aerosol generating device (104) and/or the flow stream generating element (105).

In an embodiment, the control unit (108) or the corresponding method controls the action of the skin treatment tool (103) by transmitting a trigger signal to adjust (increase or decrease) the rotational speed and/or the force imparted by the skin treatment tool, e.g., by clamping elements disposed therein, in the case of a mechanical epilator. In an embodiment, the control unit (108) or the corresponding method provides an indication to the user to manually adjust a treatment application parameter such as force or speed of operation.

The control unit (108) may be implemented in several ways, with software and/or a hardware, to perform the various functions described herein. The control unit (108) may include one or more microprocessors or digital signal processor (DSPs) that may be programmed using software or computer program code to perform the required functions and/or to control components of the control unit (108) to perform the required functions. The control unit (108) may be implemented as a combination of dedicated hardware to perform some functions (e.g., amplifiers, preamplifiers, analog-to-digital convertors (ADCs) and/or digital-to-analog convertors (DACs)) and a processor (e.g., one or more programmed microprocessors, controllers, microcontrollers, DSPs, and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

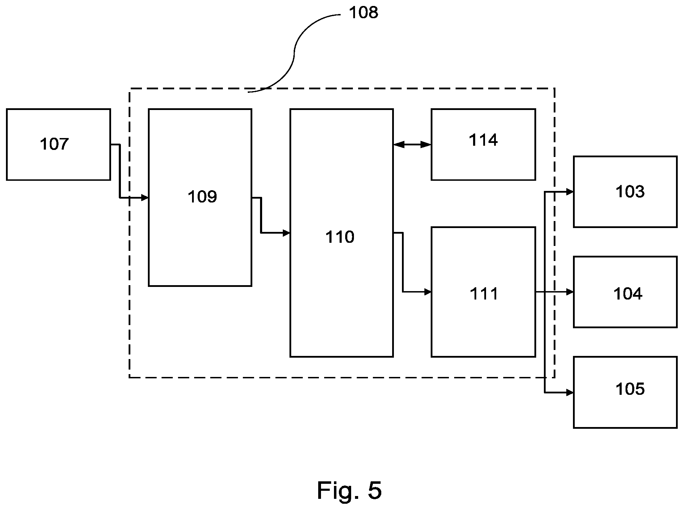

Referring to Fig. 5, the control unit (108) may include an input signal processing unit (109), a microcontroller unit (MCU) (110), an output signal processing unit (111) and a memory unit (114). The microcontroller unit (110) is connected or coupled to each of the input signal processing unit (109), the output signal processing unit (111) and the memory unit (114). The input signal processing unit (109) may receive an input signal in the form of voltage signals, perform analog-to-digital conversion of the voltage signals and may send the digitised signals to the MCU (110). In particular, the input signal processing unit (109) may receive respective operating parameter signals from the operating parameter sensor (107). The MCU (110) may perform logical operations and sends data to and receive data from the memory unit (114) and sends output signals to the output signal processing unit (111). The output signals sent to the output signal processing unit (111) may include a signal to control the operation of the skin treatment device (100).

In an embodiment, the memory unit (114) may store data, information and/or signals for use by the control unit (108) in controlling the operation of the skin treatment device (100) [thus, controlling the aerosol generating device (104)], the flow stream generating element (105) and the skin treatment tool (103) and/or in executing or performing the operations described herein. In some implementations the memory unit (114) stores computer-readable code that can be executed by the control unit (108) so that the control unit (108) performs one or more functions, including the operations described herein. The memory unit (114) may include any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In an embodiment, the MCU (110) and the output signal processing unit (111) may output a user indicator signal that indicates the status of the treatment operation to the user (or subject), for example indicating whether the skin treatment operation is efficient, based on operating parameter determined by the operating parameter sensor (107). The user indicator signal may be used to control or drive a user interface component, such as a light, display screen, loudspeaker or haptic/tactile (e.g., vibrating) element. The indication signal generated by the control unit (108) may correspond to the skin treatment device (100) being pushed hard against the skin or the skin treatment device (100) being not displaced/moved over the skin at prescribed speed, which results in generation of heat, thereby increasing temperature or changing other properties of the skin causing inconvenience to the subject and decreasing operating efficiency of the skin treatment device (100).

In an operational embodiment, i.e., during use of the skin treatment device (100) for performing skin treatment such as hair removal or hair reduction, the skin treatment device (100) may be traversed on the skin, with the skin treatment tool (103) contacting the skin. The operational embodiment hereinafter is described considering skin temperature to be the operating parameter. However, the same cannot be construed as a limitation since, the operation skin treatment operation may be performed considering other operating parameters of skin such as optical property, acoustic property, and electric property of the skin before and after the treatment. While traversing the skin treatment device (100) relative to skin of the subject, the operating parameter sensor (107) (i.e., skin temperature determining sensors) may determine temperature of the skin prior to skin treatment and temperature of the skin after subjecting to skin treatment and generate a first signal. The first signal may be received by the input signal processing unit (109), which may convert the signals into suitable form as described above. The converted signals may be fed into the MCU (110), which may analyse the signals to determine a difference in temperature of the skin before and after the skin treatment. Further, upon analysing the difference in temperature of the skin, the control unit (108) may compare the determined value with the predetermined value stored in the memory unit (114). Upon comparison, if the difference in temperature exceeds beyond a predetermined threshold value, the MCU (110) may generate a signal, which may be fed into the output signal processing unit (111). This signal may correspond to controlling operation of the aerosol generating device (104) and the flow stream generating element (105), and controlling rotational speed (i.e., RPM) of the skin treatment tool (103) in one or more manners described above. In particular, the signal fed into the output signal processing unit (111) may correspond to interrupting or varying voltage supply to the aerosol generating device (104), the flow stream generating element (105) and the skin treatment tool (103), for controlling operation of the aerosol generating device (104), the flow stream generating element (105) and the skin treatment tool (103). Such a configuration of the skin treatment device (100), where the operation of the skin treatment device (100) is controlled based on operating parameters determined by the operating parameter sensor (107), aids in increasing operational efficiency (i.e., longer operational time) of the skin treatment device (100).

In an embodiment, if the difference in temperature of the skin before and after skin treatment is within the threshold limit, the skin treatment may further operate under normal operating conditions. Normal operating conditions may infer to the aerosol generating device (104), the flow stream generating element (105) and the skin treatment tool (103) being operated in accordance to their capacity.

In some embodiments, the input signal processing unit (109) may also receive second signals from the humidity sensors. The MCU (110) may analyse the second signal and if the value of the second signal exceeds the threshold value, the output signal processing unit (111) may generate a signal, which may correspond to activating the flow stream generating element (105), independent to the aerosol generating device (104). Here, the output signal processing unit (111) may generate a signal to reduce or stop the action of the aerosol generating device (104). Activating the flow stream generating element (105), aids in reducing the humidity by forcing dry air onto the skin, which may further aid in efficient skin treatment, as excess humidity may cause damping of skin and leading to inefficient skin treatment.

In some embodiments, the control unit (108) may determine feedback for the user on the use of the skin treatment device (100) based on the determined information on the speed and/or displacement of the skin treatment device (100). The feedback may be, for example, that the user should move the skin treatment device (100) more quickly or reduce pushing force onto the skin, to improve the efficiency of the overall treatment operation.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to inventions containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean *at least* the recited number (e.g., the bare recitation of "two recitations," without other modifiers, typically means *at least* two recitations, or *two or more* recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances, where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B." While various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims. For example, although the example of hair removal is described in the description, the present invention may be applied to other kinds of skin treatment which may benefit from skin cooling - such as hair coloring, tatooing, skin rejuvenation, skin cell rejeneration etc.

### Referral Numerals:

| Reference Number | Description |
|---|---|
| 100 | Skin treatment device |
| 101 | Head |
| 102 | Handle |
| 103 | Skin treatment tool |
| 104 | Aerosol generating device |
| 105 | Flow stream generating element |
| 106 | Flow channel |
| 107 | Operating parameter sensor |
| 108 | Control unit |
| 109 | Input signal processing unit |
| 110 | Microcontroller unit |
| 111 | Output signal processing unit |
| 112 | User control |
| 114 | Memory unit |

## Claims

1. A skin treatment device (100), comprising:
a head (101);
an aerosol generating device (104);
an operating parameter sensor (107);
wherein, the aerosol generating device (104) is arranged to generate and channelize an aerosol through a flow channel (106) of the skin treatment device (100) out of the skin treatment device (100) and onto skin subjected to treatment to provide a cooling effect to the skin, based on an operating parameter measured by the operating parameter sensor (107);
**characterized in that** the aerosol generating device (104) is disposed on the head (101) and parallel to a longitudinal axis (A-A) of the skin treatment device (100).

2. The skin treatment device (100) as claimed in claim 1, further comprising a flow stream generating element, configured to generate a flow to further channelize the aerosol through the flow channel (106).

3. The skin treatment device (100) as claimed in claim 2, wherein the flow stream generating element is at least one of a blower and a suction fan.

4. The skin treatment device (100) as claimed in any of claims 1-3, wherein the flow channel (106) is defined by spacings in a skin treatment tool (103) of the skin treatment device (100).

5. The skin treatment device (100) as claimed in any of claims 1-4, wherein the flow channel (106) is a passage defined in or coupled to the skin treatment device (100).

6. The skin treatment device (100) as claimed in any of the preceding claims, wherein the operating parameter sensor (107) is an optical sensor.

7. The skin treatment device (100) as claimed in any of the preceding claims, wherein the operating parameter sensor (107) is configured to measure at least one of a skin parameter and a skin treatment device parameter.

8. The skin treatment device (100) as claimed in claim 7, wherein the skin parameter is at least one of temperature of skin, humidity of skin, and skin tone.

9. The skin treatment device (100) as claimed in claim 7, wherein the skin treatment device parameter is at least one of temperature, mechanical force, and displacement of a skin treatment tool (103).

10. The skin treatment device (100) as claimed in any of the preceding claims, further comprising a plurality of operating parameter sensors, wherein each of the plurality of operating parameter sensors are disposed on opposite sides relative to a longitudinal axis (B-B) of a skin treatment tool (103) of the skin treatment device (100).

11. The skin treatment device (100) as claimed in any of the preceding claims, wherein the aerosol generating device (104) is a piezo-electric device.

12. The skin treatment device (100) as claimed in any of the preceding claims, further comprising a control unit (108) communicatively coupled to the operating parameter sensor (107) and the aerosol generating device (104), wherein the control unit (108) is configured to selectively control operation of the aerosol generating device (104) and/or a skin treatment tool (103) of the skin treatment device (100) based on the operating parameter measured by the operating parameter sensor (107).

13. The skin treatment device (100) as claimed in claim 12, with claim 12 being dependent on claim 2 or 3, wherein the control unit (108) is communicatively coupled to the flow stream generating element and configured to actuate the flow stream generating element independent of the aerosol generating device (104) based on humidity of the skin measured by the operating parameter sensor (107).

14. The skin treatment device (100) as claimed in any of the preceding claims, further comprising an indication unit communicatively coupled to a control unit (108), wherein the control unit (108) is configured to generate an alert signal through the indication unit based on signals from the operating parameter sensor (107), and/or wherein the alert signal is at least one of an audio signal, a visual signal, an audio-visual signal, and a haptic signal.

## Patentansprüche

1. Hautbehandlungsvorrichtung (100) umfassend:
einen Kopf (101);
eine Aerosolerzeugungsvorrichtung (104);
einen Betriebsparametersensor (107);
wobei die Aerosolerzeugungsvorrichtung (104) angeordnet ist, um ein Aerosol zu erzeugen und durch einen Strömungskanal (106) der Hautbehandlungsvorrichtung (100) aus der Hautbehandlungsvorrichtung (100) heraus und auf die zu behandelnde Haut zu kanalisieren, um der Haut basierend auf einem vom Betriebsparametersensor (107) gemessenen Betriebsparameter einen Kühleffekt bereitzustellen;
**dadurch gekennzeichnet, dass** die Aerosolerzeugungsvorrichtung (104) am Kopf (101) und parallel zu einer Längsachse (A-A) der Hautbehandlungsvorrichtung (100) angeordnet ist.

2. Hautbehandlungsvorrichtung (100) nach Anspruch 1, die weiter ein Strömungserzeugungselement umfasst, das konfiguriert ist, um eine Strömung zu erzeugen, um das Aerosol weiter durch den Strömungskanal (106) zu kanalisieren.

3. Hautbehandlungsvorrichtung (100) nach Anspruch 2, wobei das Strömungserzeugungselement mindestens einer von einem Gebläse und einem Sauglüfter ist.

4. Hautbehandlungsvorrichtung (100) nach einem der Ansprüche 1-3, wobei der Strömungskanal (106) durch Zwischenräume in einem Hautbehandlungswerkzeug (103) der Hautbehandlungsvorrichtung (100) definiert ist.

5. Hautbehandlungsvorrichtung (100) nach einem der Ansprüche 1-4, wobei der Strömungskanal (106) ein Durchgang ist, der in der Hautbehandlungsvorrichtung (100) definiert ist oder damit gekoppelt ist.

6. Hautbehandlungsvorrichtung (100) nach einem der vorstehenden Ansprüche, wobei der Betriebsparametersensor (107) ein optischer Sensor ist.

7. Hautbehandlungsvorrichtung (100) nach einem der vorstehenden Ansprüche, wobei der Betriebsparametersensor (107) konfiguriert ist, um mindestens einen von einem Hautparameter und einem Parameter der Hautbehandlungsvorrichtung zu messen.

8. Hautbehandlungsvorrichtung (100) nach Anspruch 7, wobei der Hautparameter mindestens einer von Hauttemperatur, Hautfeuchtigkeit und Hautton ist.

9. Hautbehandlungsvorrichtung (100) nach Anspruch 7, wobei der Parameter der Hautbehandlungsvorrichtung mindestens einer von Temperatur, mechanischer Kraft und Verschiebung eines Hautbehandlungswerkzeugs (103) ist.

10. Hautbehandlungsvorrichtung (100) nach einem der vorstehenden Ansprüche, die weiter eine Vielzahl von Betriebsparametersensoren umfasst, wobei jeder der Vielzahl von Betriebsparametersensoren auf gegenüberliegenden Seiten in Bezug zu einer Längsachse (B-B) eines Hautbehandlungswerkzeugs (103) der Hautbehandlungsvorrichtung (100) angeordnet ist.

11. Hautbehandlungsvorrichtung (100) nach einem der vorstehenden Ansprüche, wobei die Aerosolerzeugungsvorrichtung (104) eine piezoelektrische Vorrichtung ist.

12. Hautbehandlungsvorrichtung (100) nach einem der vorstehenden Ansprüche, die weiter eine Steuereinheit (108) umfasst, die kommunikativ mit dem Betriebsparametersensor (107) und der Aerosolerzeugungsvorrichtung (104) gekoppelt ist, wobei die Steuereinheit (108) konfiguriert ist, um selektiv einen Betrieb der Aerosolerzeugungsvorrichtung (104) und/oder eines Hautbehandlungswerkzeugs (103) der Hautbehandlungsvorrichtung (100) basierend auf dem vom Betriebsparametersensor (107) gemessenen Betriebsparameter zu steuern.

13. Hautbehandlungsvorrichtung (100) nach Anspruch 12, wobei Anspruch 12 von Anspruch 2 oder 3 abhängig ist, wobei die Steuereinheit (108) kommunikativ mit dem Strömungserzeugungselement gekoppelt ist und konfiguriert ist, um das Strömungserzeugungselement unabhängig von der Aerosolerzeugungsvorrichtung (104) basierend auf der vom Betriebsparametersensor (107) gemessenen Feuchtigkeit der Haut zu betätigen.

14. Hautbehandlungsvorrichtung (100) nach einem der vorstehenden Ansprüche, die weiter eine Anzeigeeinheit umfasst, die kommunikativ mit einer Steuereinheit (108) gekoppelt ist, wobei die Steuereinheit (108) konfiguriert ist, um basierend auf Signalen vom Betriebsparametersensor (107) ein Warnsignal über die Anzeigeeinheit zu erzeugen, und/oder wobei das Warnsignal mindestens eines von einem Audiosignal, einem visuellen Signal, einem audiovisuellen Signal und einem haptischen Signal ist.

## Revendications

1. Dispositif de traitement de la peau (100), comprenant :
une tête (101) ;
un dispositif de génération d'aérosol (104) ;
un capteur de paramètres de fonctionnement (107) ;
dans lequel le dispositif de génération d'aérosol (104) est agencé pour générer et canaliser un aérosol à travers un canal d'écoulement (106) du dispositif de traitement de la peau (100) à partir du dispositif de traitement de la peau (100) et sur une peau soumise à un traitement de façon à produire un effet de refroidissement sur la peau, sur la base d'un paramètre de fonctionnement mesuré par le capteur de paramètres de fonctionnement (107) ;
**caractérisé en ce que** le dispositif de génération d'aérosol (104) est disposé sur la tête (101) et parallèlement à un axe longitudinal (A-A) du dispositif de traitement de la peau (100).

2. Dispositif de traitement de la peau (100) selon la revendication 1, comprenant en outre un élément de génération de flux d'écoulement configuré pour générer un écoulement de façon à canaliser davantage l'aérosol à travers le canal d'écoulement (106).

3. Dispositif de traitement de la peau (100) selon la revendication 2, dans lequel l'élément de génération de flux d'écoulement est au moins l'un parmi un ventilateur soufflant et un ventilateur aspirant.

4. Dispositif de traitement de la peau (100) selon l'une quelconque des revendications 1-3, dans lequel le canal d'écoulement (106) est défini par des espacements dans un outil de traitement de la peau (103) du dispositif de traitement de la peau (100).

5. Dispositif de traitement de la peau (100) selon l'une quelconque des revendications 1-4, dans lequel le canal d'écoulement (106) est un passage défini dans ou couplé au dispositif de traitement de la peau (100).

6. Dispositif de traitement de la peau (100) selon l'une quelconque des revendications précédentes, dans lequel le capteur de paramètres de fonctionnement (107) est un capteur optique.

7. Dispositif de traitement de la peau (100) selon l'une quelconque des revendications précédentes, dans lequel le capteur de paramètres de fonctionnement (107) est configuré pour mesurer au moins l'un parmi un paramètre de la peau et un paramètre du dispositif de traitement de la peau.

8. Dispositif de traitement de la peau (100) selon la revendication 7, dans lequel le paramètre de la peau est au moins l'un parmi la température de la peau, l'humidité de la peau et le teint de la peau.

9. Dispositif de traitement de la peau (100) selon la revendication 7, dans lequel le paramètre du dispositif de traitement de la peau est au moins l'un parmi la température, la force mécanique et le déplacement d'un outil de traitement de la peau (103).

10. Dispositif de traitement de la peau (100) selon l'une quelconque des revendications précédentes, comprenant en outre une pluralité de capteurs de paramètres de fonctionnement, dans lequel chacun de la pluralité de capteurs de paramètres de fonctionnement est disposé sur des côtés opposés par rapport à un axe longitudinal (B-B) d'un outil de traitement de la peau (103) du dispositif de traitement de la peau (100).

11. Dispositif de traitement de la peau (100) selon l'une quelconque des revendications précédentes, dans lequel le dispositif de génération d'aérosol (104) est un dispositif piézoélectrique.

12. Dispositif de traitement de la peau (100) selon l'une quelconque des revendications précédentes, comprenant en outre une unité de commande (108) couplée en communication au capteur de paramètres de fonctionnement (107) et au dispositif de génération d'aérosol (104), dans lequel l'unité de commande (108) est configurée pour commander sélectivement le fonctionnement du dispositif de génération d'aérosol (104) et/ou d'un outil de traitement de la peau (103) du dispositif de traitement de la peau (100) sur la base du paramètre de fonctionnement mesuré par le capteur de paramètres de fonctionnement (107).

13. Dispositif de traitement de la peau (100) selon la revendication 12, la revendication 12 dépendant de la revendication 2 ou 3, dans lequel l'unité de commande (108) est couplée en communication à l'élément de génération de flux d'écoulement et configurée pour actionner l'élément de génération de flux d'écoulement indépendamment du dispositif de génération d'aérosol (104) sur la base de l'humidité de la peau mesurée par le capteur de paramètres de fonctionnement (107).

14. Dispositif de traitement de la peau (100) selon l'une quelconque des revendications précédentes, comprenant en outre une unité d'indication couplée en communication à une unité de commande (108), dans lequel l'unité de commande (108) est configurée pour générer un signal d'alerte par l'intermédiaire de l'unité d'indication sur la base de signaux provenant du capteur de paramètres de fonctionnement (107) et/ou dans lequel le signal d'alerte est au moins l'un parmi un signal audio, un signal visuel, un signal audiovisuel et un signal haptique.
